# EUROPÄISCHE PATENTSCHRIFT

(11) **EP 1 078 918 B1**
(45) Veröffentlichungstag und Bekanntmachung des Hinweises auf die Patenterteilung: **11.12.2002**
(21) Anmeldenummer: 00202871.0
(22) Anmeldetag: 29.03.1995
(51) Int. Cl.: C07C 263/10, C07C 265/10

(54) **Verfahren zur Herstellung von Diisocyanaten**
Process for the preparation of diisocyanates
Procédé pour la préparation des diisocyanates

(30) Priorität: 11.04.1994 DE 4412327
(43) Veröffentlichungstag der Anmeldung: 28.02.2001
(62) Teilanmeldung aus: 95104637.4
(73) Patentinhaber: BAYER AG, 51368 Leverkusen (DE)
(72) Erfinder: Bischof, Eric, Dr., 42799 Leichlingen (DE); Breidenbach, Peter, Dr., 50735 Köln (DE); Dahmer, Jürgen, Dr., 47800 Krefeld (DE); Flink, Andreas, 41539 Dormagen (DE); Molnar, Attila, Dr., 51519 Odenthal (DE); Stutz, Herbert, Dr., 41541 Dormagen (DE)

(56) Entgegenhaltungen:
- EP-A- 0 289 840
- GB-A- 1 173 890
- US-A- 3 644 459
- Research Disclosure, Nr. 335052 (1992)

## Beschreibung

Die Erfindung betrifft ein Verfahren zur Herstellung von nicht in technisch relevanter Ausbeute verfügbaren cycloaliphatischen Diisocyanaten mit Isocyanatgruppen in 1,2-oder 1,3-Stellung zueinander durch Phosgenierung der entsprechenden Diamine in der Gasphase.

Obwohl die Herstellung von organischen Isocyanaten durch Umsetzung von Aminen mit Phosgen in der Gasphase seit langem bekannt ist (z.B. Siefken, Justus Liebigs Anm. Chem. 562, 108 (1949)), wurde es bislang nur zur Herstellung von Monoisocyanaten (z.B. Ullmann, 4. Auflage, Band 13, Seite 353), von technisch bereits verfügbaren (cyclo)aliphatischen Diisocyanaten (EP-A-0 289 840) oder zur Herstellung der großtechnischen aromatischen Diisocyanate (DE-OS 4 217 019) empfohlen.

Obwohl cycloaliphatische 1,2- und 1,3-Diisocyanate in der Literatur vielfach Erwähnung finden, muss darauf verwiesen werden, dass diese Diisocyanate keine in technischen Mengen zur Verfügung stehenden Verbindungen darstellen. Diese Diisocyanate sind nämlich durch die klassische Phosgenierung der entsprechenden Diamine in der Flüssigphase nicht in technisch vertretbaren Ausbeuten zugänglich. Ursache hierfür sind neben der niedrigen Rohwareausbeute die starken Verluste während der Aufarbeitung, die in einigen Fällen nur ein Isolieren von 30 % des ursprünglich gebildeten Isocyanats erlauben.

So liefert die herkömmliche Phosgenierung von 1,3-Diaminopentan in der Flüssigphase Rohwareausbeuten in der Größenordnung von 30 % und isolierte Ausbeuten in der Größenordnung von 10 % bezogen auf eingesetztes Amin (E.I. DuPont and Co., Res. Discl., 335, 195 von 1992 und eigene Arbeiten). Nachteilig sind' die erforderlichen langen Heißphosgenierzeiten und hohen Phosgenüberschüsse sowie die extrem niedrige Ausbeute.

Die Phosgenierung von 2,4(6)-Diamino-1-methylcyclohexan in der Flüssigphase ergibt in der Regel nur unbefriedigende Ausbeuten, obwohl in der DE-OS 2 005 309 bzw. in der BE-PS 745 440 Ausbeuten von bis zu 89 % bzw. von 92 % beschrieben sind. Bei der Aufarbeitung der gemäß diesen Vorveröffentlichungen gewonnenen Rohware treten jedoch zusätzliche Ausbeuteverluste auf.

Wie jetzt überraschenderweise gefunden wurde, gelingt es, die genannten Diisocyanate durch Phosgenierung der entsprechenden Diamine in ganz wesentlich erhöhten Ausbeuten unter Eliminierung der genannten Nachteile des Standes der Technik herzustellen, wenn die Phosgenierung der Diamine und die anschließende Aufarbeitung des Reaktionsgemischs in Analogie zur Verfahrensweise der EP-A 0 289 840 erfolgt. Eine derartige ganz wesentliche Ausbeuteverbesserung konnte nicht erwartet werden, da die schlechten Ausbeuten der Verfahren des Standes der Technik insbesondere auf Nebenreaktionen wie beispielsweise die Bildung von cyclischen Harnstoffderivaten während der Phosgenierungsreaktion zurückzuführen ist, von denen nicht vorhersehbar war, dass sie bei der Gasphasenphosgenierung weitgehend unterbleiben.

Gegenstand der Erfindung ist ein Verfahren zur Herstellung von Diisocyanaten durch Phosgenierung der entsprechenden Diamine in der Gasphase, indem man
a) die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und kontinuierlich in einem auf 200°C bis 600°C erhitzten, zylindrischen Reaktionsraum ohne sich bewegende Teile unter Aufrechterhaltung einer turbulenten Strömung im Reaktionsraum miteinander zur Reaktion bringt,
b) das den Reaktionsraum kontinuierlich verlassende Gasgemisch mit Hilfe eines inerten, flüssigen Lösungsmittels, welches bei einer Temperatur oberhalb der Zersetzungstemperatur des dem Diamin entsprechenden Carbamidsäurechlorid gehalten wird unter Gewinnung einer Lösung des Diisocyanats in diesem Lösungsmittel abkühlt und
c) das in dem inerten Lösungsmittel gelöst vorliegende Diisocyanat einer destillativen Aufarbeitung unterzieht,
dadurch gekennzeichnet, dass man als zu phosgenierende Diamine solche verwendet, die aus der Gruppe bestehend aus cycloaliphatischen Diaminen mit zwei primären Aminogruppen in 1,2- oder 1,3-Stellung zueinander ausgewählt worden sind.

Das erfindungsgemäße Verfahren eignet sich zur Phosgenierung von beliebigen cycloaliphatischen Diaminen mit primären Aminogruppen in 1,2- oder 1,3-Stellung, vorausgesetzt, die Diamine sind unter den Temperaturbedingungen des erfindungsgemäßen Verfahrens stabil und gasförmig. Besonders bevorzugt werden als Ausgangsmaterialien für das erfindungsgemäße Verfahren diprimäre cycloaliphatische Diamine mit 6 bis 15, vorzugsweise 6 bis 12 Kohlenstoffatomen und zwei Aminogruppen in 1,2- oder 1,3-Stellung eingesetzt.

Zu den geeigneten bzw. bevorzugt geeigneten Diaminen gehören beispielsweise 2,3-, 2,4- und 2,6-Diamino-1-methylcyclohexan, Gemische von mindestens zwei der drei letztgenannten Diamine, 1,2-Diaminocyclohexan, 1-Methyl-3,5-diethyl-2,4-diaminocyclohexan, 1-Methyl-3,5-diethyl-2,6-diaminocyclohexan, Gemische der beiden letztgenannten Diamine, 1,3,5-Triisopropyl-2,4-diaminocyclohexan, 2,4-Diamino-1-isopropylcyclohexan, 2,6-Diamino-1-isopropylcyclohexan oder Gemische der beiden letztgenannten Diamine.

Die Ausgangsdiamine werden vor der Durchführung des erfindungsgemäßen Verfahrens verdampft und kontinuierlich auf eine Temperatur innerhalb des Temperaturbereichs von 200 bis 600°C, vorzugsweise 300 bis 500°C erhitzt. Die zuvor erhitzten Diamindämpfe können beim erfindungsgemäßen Verfahren als solche oder in mit Inertgas oder mit Dämpfen eines inerten Lösungsmittels verdünnter Form zum Einsatz gelangen. Die Durchmischung der Diamindämpfe mit dem Inertgas kann beispielsweise durch Verdampfen des Diamins im Strom eines inerten Gases oder der Dämpfe eines inerten Lösungsmittels erfolgen. Bevorzugtes Inertgas ist Stickstoff. Geeignete inerte Lösungsmittel, deren Dämpfe ebenfalls zur Verdünnung des Diamins verwendet werden können sind beispielsweise Chlorbenzol, o-Dichlorbenzol, Xylol, Chlornaphthalin, Decahydronaphthalin oder deren Gemische.

Die Menge des gegebenenfalls als Verdünnungsmittel mitverwendeten Inertgases oder Lösungsmitteldampfes ist nicht kritisch.

Das bei der Phosgenierung verwendete Phosgen wird, bezogen auf das Diamin, im Überschuss eingesetzt. Im allgemeinen genügt eine Phosgenmenge, die 150 bis 300 % der Theorie bezüglich der ablaufenden Phosgenierungsreaktion entspricht.

Vor der Durchführung des erfindungsgemäßen 'Verfahrens wird der Phosgenstrom auf eine Temperatur innerhalb des Bereichs von 200 bis 600°C, vorzugsweise 300 bis 500°C erhitzt.

Zur Durchführung der erfindungsgemäßen Umsetzung werden die vorerhitzten Ströme von Diamin bzw. Diamin-Inertgas-Gemisch einerseits und von Phosgen andererseits kontinuierlich in einen zylindrischen Reaktionsraum geleitet und dort miteinander vermischt.

Geeignete zylinderförmige Reaktionsräume sind beispielsweise Rohrreaktoren ohne Einbauten und ohne sich bewegende Teile im Innern des Reaktors. Die Rohrreaktoren bestehen im allgemeinen aus Stahl, Glas, legiertem oder emailliertem Stahl und weisen eine Länge auf, die ausreichend ist, um unter den Verfahrensbedingungen eine vollständige Umsetzung des Diamins mit dem Phosgen zu ermöglichen. Die Gasströme werden im allgemeinen an einem Ende des Rohrreaktors in diesen eingeleitet, wobei diese Einleitung beispielsweise durch an einem Ende des Rohrreaktors angebrachte Düsen oder durch eine Kombination aus Düse und einem Ringspalt zwischen Düse und Mischrohr erfolgen kann. Das Mischrohr wird ebenfalls bei einer Temperatur innerhalb des Bereichs von 200 bis 600°C, vorzugsweise 300 bis 500°C gehalten, wobei diese Temperatur gegebenenfalls durch Beheizen des Reaktionsrohrs aufrechterhalten wird.

Für die Durchführbarkeit des erfindungsgemäßen Verfahrens wesentlich ist, dass die Abmessungen des Rohrreaktors und die Strömungsgeschwindigkeiten im Reaktionsraum so bemessen werden, dass in dem Reaktionsraum eine turbulente Strömung herrscht. Unter "turbulenter Strömung" ist hierbei eine Reynoldszahl von mindestens 2500, vorzugsweise mindestens 4700 zu verstehen. Im allgemeinen ist diese Turbulenz im Reaktionsraum dann gewährleistet, wenn die gasförmigen Reaktionspartner mit einer Strömungsgeschwindigkeit von mehr als 90 m/s den Reaktionsraum durchlaufen. Diese Strömungsgeschwindigkeit kann durch Einstellung eines entsprechenden Differenzdrucks zwischen den Produktzuleitungen zum Reaktionsraum einerseits und Ausgang aus dem Reaktionsraum andererseits sichergestellt werden. Im allgemeinen liegt der Druck in den Zuleitungen zum Reaktionsraum bei 200 bis 3000 mbar und am Ausgang aus dem Reaktionsraum bei 150 bis 2000 mbar. Wesentlich ist hierbei jedoch lediglich die Aufrechterhaltung eines Differenzdrucks zwecks Gewährleistung der genannten Strömungsgeschwindigkeit.

Nach der erfolgten Phosgenierungsreaktion im Reaktionsraum wird das den Reaktionsraum kontinuierlich verlassende, gasförmige Gemisch von dem gebildeten Diisocyanat befreit. Dies kann beispielsweise mit Hilfe eines inerten Lösungsmittels erfolgen, dessen Temperatur so gewählt wird, dass sie einerseits oberhalb der Zersetzungstemperatur des dem Diisocyanat entsprechenden Carbamidsäurechlorids liegt und andererseits das Diisocyanat und gegebenenfalls das in der Dampfform als Verdünnungsmittel mitverwendete Lösungsmittel kondensieren bzw. sich in dem Lösungsmittel lösen, während überschüssiges Phosgen, Chlorwasserstoff und gegebenenfalls als Verdünnungsmittel mitverwendetes Inertgas die Kondensationsstufe bzw. das Lösungsmittel gasförmig durchlaufen. Zur selektiven Gewinnung des Diisocyanats aus dem den Reaktionsraum gasförmig verlassenden Gemisch besonders gut geeignet sind bei einer Temperatur von 120 bis 200°C, vorzugsweise 120 bis 170°C gehaltene Lösungsmittel der oben beispielhaft genannten Art insbesondere technisches Dichlorbenzol. Denkbare Methoden der selektiven Kondensation des gebildeten Diisocyanats aus dem den Reaktor verlassenden Gasgemisch unter Verwendung derartiger Lösungsmittel sind beispielsweise das Durchleiten des Gasgemisches durch das genannte Lösungsmittel oder das Eindüsen des Lösungsmittels (Lösungsmittelnebel) in den Gasstrom.

Das die Kondensationsstufe zur Gewinnung des Diisocyanats durchlaufende Gasgemisch wird anschließend in an sich bekannter Weise von überschüssigem Phosgen befreit. Dies kann mittels einer Kühlfalle, Absorption in einem bei einer Temperatur von -10°C bis 8°C gehaltenen inerten Lösungsmittel (z.B. Chlorbenzol oder Dichlorbenzol) oder durch Adsorption und Hydrolyse an Aktivkohle erfolgen. Das die Phosgen-Rückgewinnungsstufe durchlaufende Chlorwasserstoffgas kann in an sich bekannter Weise zur Rückgewinnung des zur Phosgensynthese erforderlichen Chlors recyclisiert werden.

Die Reindarstellung der Diisocyanate erfolgt durch destillative Aufarbeitung der Lösung des Diisocyanats in dem zur Diisocyanatkondensation eingesetzten Lösungsmittel. In den nachfolgenden Beispielen beziehen sich alle Prozentangaben auf Gewichtsprozente. In allen Beispielen bewirken die zur Anwendung gelangenden Strömungsverhältnisse im Reaktionsraum Turbulenzen, die Reynolds-Zahlen von mindestens 2500 entsprechen.

### Beispiel 1 (1,3-Pentandiisocyanat) (gehört zur Stammanmeldung, Anmelde-Nummer 95 104 637.4)

In ein auf 400°C erwärmtes Mischrohr von 2,5 mm Durchmesser und 17,5 mm Länge mit nachgeschalteter Diisocyanatkondensationsstufe und dieser nachfolgendem, mit Aktivkohle gefülltem Phosgen-Adsorptionssturm, strömen kontinuierlich durch die Düse, die in das Mischrohr hineinragt, 5,9 Mol/Stunde Phosgen, die in einem vorgeschalteten Wärmetauscher bei einem Druck von 950 mbar auf eine Temperatur von 420°C erwärmt wurden. Durch den Ringspalt zwischen Düse und Mischrohr wird gleichzeitig ein auf 250°C erwärmtes Gemisch aus 1 Mol/Stunde 1,3-Diaminopentan und 0,1 Mol/Stunde Stickstoff als Verdünnungsmittel in das Mischrohr geleitet. Durch Anlegen eines Vakuums am Ende der Diisocyanatkondensationsstufe wird ein Druck im Mischrohr von ca. 350 mbar aufrechterhalten. Das heiße, den Reaktionsraum gasförmig verlassende Reaktionsgemisch wird in der Kondensationsstufe durch Dichlorbenzol geleitet, welches bei einer Temperatur von 150 bis 160°C gehalten wird. Hierbei erfolgt die selektive Kondensation des gebildeten Diisocyanats. Das die Waschstufe durchlaufende, im wesentlichen aus Stickstoff, Chlorwasserstoff und überschüssigem Phosgen bestehende Gasgemisch wird in dem Adsorptionssturm anschließend vom Phosgen befreit. Das Diisocyanat wird aus dem Waschlösungsmittel destillativ in reiner Form gewonnen. Die Ausbeute an reinem 1,3-Pentandiisocyanat liegt bei 80 % der Theorie.

### Vergleichsbeispiel (Flüssigphasenphosgenierung von 1,3-Diaminopentan)

In ein Gemisch bestehend aus 38 g 1,3-Diaminopentan und 1000 g 1,2-Dichlorbenzol wird bis zur Sättigung CO₂ eingeleitet. Das so erhaltene Carbaminat-Lösungsmittel-Gemisch wird nun über einen Zeitraum von ca. 1 Stunde zu einer Lösung von 220 g Phosgen in 100 g 1,2-Dichlorbenol, welches bei ca. 0°C gehalten wird, getropft. Anschließend wird das Reaktionsgemisch unter kontinuierlichem Durchleiten von 24 l/h Phosgengas auf 170°C (Siedetemperatur des Lösungsmittels) erwärmt und 4 Stunden bei dieser Temperatur gehalten. Das so erhaltene Reaktionsgemisch enthält 25 % der Theorie an 1,3-Diisocyanatopentan. Nach destillativer Aufarbeitung wurde das 1,3-Diisocyanatopentan in 93%iger Reinheit mit einer Ausbeute von 17% der Theorie gewonnen.

### Beispiel 2 (2,4(6)-Diisocyanato-1-methylcyclohexan)

Unter den Verfahrensbedingungen des Beispiels 1 werden 1 Mol/Stunde eines Isomerengemisches basierend auf 65 % 2,4-Diamino-1-methylcyclohexan und 35 % 2,6-Diamino-1-methylcyclohexan mit 5,9 Mol/Stunde Phosgen unter Mitverwendung von 0,1 Mol/Stunde Stickstoff als Verdünnungsmittel für das Amin in 98%iger Ausbeute in das 2,4(6)-Diisocyanato-1-methylcyclohexan überführt. Die destillative Aufarbeitung gelingt ohne Probleme und Liefert 96 % der Theorie reines Diisocyanat.

### Beispiel 3 (3,5-Diethyl-2,4(6)-diisocyanato-1-methylcyclohexan)

Unter den Verfahrensbedingungen des Beispiels 1 werden 1 Mol/Stunde eines Isomerengemisches von 2,4(6)-Diamino-3,5-diethyl-1-methylcyclohexan mit 5,9 Mol/Stunde Phosgen unter Mitverwendung von 0,1 Mol/Stunde Stickstoff als Verdünnungsmittel für das Amin in 99 %iger Ausbeute in das 3,5-Diethyl-2,4(6)-diisocyanato-1-methylcyclohexan überführt. Die destillative Aufarbeitung gelingt ohne Probleme und liefert 98 % der Theorie reines Diisocyanat.

### Beispiel 4 (1,2-Diisocyanatocyclohexan)

Unter den Verfahrensbedingungen des Beispiels 1 werden 1 Mol/Stunde eines cis/trans-Isomerengemisches von 1,2-Diaminocyclohexan mit 5,9 Mol/Stunde Phosgen unter Mitverwendung von 0,1 Mol/Stunde Stickstoff als Verdünnungsmittel für das Amin in 52 %iger Ausbeute in das 1,2-Diisocyanatocyclohexan überführt. Die destillative Aufarbeitung gelingt ohne Probleme und liefert 42 % der Theorie reines Diisocyanat.

## Patentansprüche

1. Verfahren zur Herstellung von Diisocyanaten durch Phosgenierung der entsprechenden Diamine in der Gasphase, indem man
a) die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und kontinuierlich in einem auf 200°C bis 600°C erhitzten, zylindrischen Reaktionsraum ohne sich bewegende Teile unter Aufrechterhaltung einer turbulenten Strömung im Reaktionsraum miteinander zur Reaktion bringt,
b) das den Reaktionsraum kontinuierlich verlassende Gasgemisch mit Hilfe eines inerten, flüssigen Lösungsmittels, welches bei einer Temperatur oberhalb der Zersetzungstemperatur des dem Diamin entsprechenden Carbamidsäurechlorid gehalten wird unter Gewinnung einer Lösung des Diisocyanats in diesem Lösungsmittel abkühlt und
c) das in dem inerten Lösungsmittel gelöst vorliegende Diisocyanat einer destillativen Aufarbeitung unterzieht,
**dadurch gekennzeichnet, dass** man als zu phosgenierende Diamine solche verwendet, die aus der Gruppe bestehend aus cycloaliphatischen Diaminen mit zwei primären Aminogruppen in 1,2- oder 1,3-Stellung zueinander ausgewählt worden sind.

2. Verfahren gemäß Anspruch 1, **dadurch gekennzeichnet, dass** man als zu phosgenierendes Diamin 2,3-, 2,4- und 2,6-Diamino-1-methylcyclohexan, Gemische von mindestens zwei der drei letztgenannten Diamine, 1,2-Diaminocyclohexan, 1-Methyl-3,5-diethyl-2,4-diaminocyclohexan, 1-Methyl-3,5-diethyl-2,6-diaminocyclohexan, Gemische der beiden letztgenannten Diamine, 1,3,5-Triisopropyl-2,4-diaminocyclohexan, 2,4-Diamino-1-isopropylcyclohexan, 2,6-Diamino-1-isopropylcyclohexan oder Gemische der beiden letztgenannten Diamine verwendet.

3. Verfahren gemäß Anspruch 1 und 2, **dadurch gekennzeichnet, dass** man die in dem Reaktionsraum vorherrschende Turbulenz einer Reynoldszahl von mindestens 2500 entspricht.

4. Verfahren gemäß Anspruch 1 bis 3, **dadurch gekennzeichnet, dass** man die Umsetzung in dem Reaktionsraum bei einer Temperatur von 300 bis 500°C durchführt.

5. Verfahren gemäß Anspruch 1 bis 4, **dadurch gekennzeichnet, dass** man als Inertgas zur Verdünnung des dampfförmigen Diamins Stickstoff verwendet.

## Claims

1. Process for the preparation of diisocyanates by phosgenation of the corresponding diamines in the gas phase, wherein
a) the vaporous diamines, optionally diluted with an inert gas or with the vapours of an inert solvent, and phosgene are heated separately to temperatures of from 200°C to 600°C and are caused to react continuously with one another in a cylindrical reaction chamber heated to 200°C to 600°C, which lacks moving parts, with a turbulent flow being maintained in the reaction chamber,
b) the gas mixture continuously leaving the reaction chamber is cooled by means of an inert, liquid solvent, which is maintained at a temperature above the decomposition temperature of the carbamic acid chloride corresponding to the diamine, with a solution of the diisocyanate in the said solvent being obtained and
c) the diisocyanate present dissolved in the inert solvent is subjected to working-up by distillation,
**characterised in that** the diamines to be phosgenated that are used are those which have been selected from among the cycloaliphatic diamines having two primary amino groups in the 1,2-position or the 1,3-position to one another.

2. Process according to claim 1, **characterised in that** the diamines to be phosgenated that are used are 2,3-, 2,4- and 2,6-diamino-1-methylcyclohexane, mixtures of at least two of the three last-named diamines, 1,2-diamino-cyclohexane, 1-methyl-3,5-diethyl-2,4-diaminocyclohexane, 1-methyl-3,5-diethyl-2,6-diaminocyclohexane, mixtures of the two last-named diamines, 1,3,5-triisopropyl-2,4-diaminocyclohexane, 2,4-diamino-1-isopropyl-cyclohexane, 2,6-diamino-1-isopropylcyclohexane or mixtures of the two last-named diamines.

3. Process according to claims 1 and 2, **characterised in that** the turbulence prevailing in the reaction chamber corresponds to a Reynolds number of at least 2500.

4. Process according to claims 1 to 3, **characterised in that** the reaction is carried out in the reaction chamber at a temperature of from 300 to 500°C.

5. Process according to claims 1 to 4, **characterised in that** nitrogen is used as the inert gas for the dilution of the vaporous diamine.

## Revendications

1. Procédé pour la préparation de diisocyanates par phosgénation des diamines correspondantes en phase gazeuse dans lequel
a) on chauffe séparément à des températures de 200 à 600°C les diamines à l'état de vapeurs, le cas échéant diluées par un gaz inerte ou par les vapeurs d'un solvant inerte, et le phosgène, et on les fait réagir en continu dans une zone de réaction cylindrique sans parties mobiles, chauffée à une température de 200 à 600°C, en maintenant dans la zone de réaction un courant turbulent,
b) on refroidit le mélange gazeux quittant en continu la zone de réaction à l'aide d'un solvant liquide inerte maintenu à une température supérieure à la température de décomposition du chlorure d'acide carbamique correspondant à la diamine, obtenant ainsi une solution du diisocyanate dans ce solvant et
c) on soumet le diisocyanate, à l'état de solution dans le solvant inerte, à un traitement de distillation,
**caractérisé en ce que** les diamines soumises à la phosgénation sont choisies dans le groupe consistant en les diamines cycloaliphatiques à deux groupes amino primaires en position mutuelle 1,2 ou 1,3.

2. Procédé selon la revendication 1, **caractérisé en ce que** la diamine soumise à la phosgénation est le 2,3-, le 2,4- ou le 2,6-diamino-1-méthylcyclohexane, un mélange d'au moins deux de ces trois diamines, le 1,2-diaminocyclohexane, le 1-méthyl-3,5-diéthyl-2,4-diaminocyclohexane, le 1-méthyl-3,5-diéthyl-2,6-diaminocyclohexane, un mélange des deux diamines citées en dernier, le 1,3,5-triisopropyl-2,4-diaminocyclohexane, le 2,4-diamino-1-isopropylcyclohexane, le 2,6-diamino-1-isopropylcyclohexane ou un mélange de ces deux dernières diamines.

3. Procédé selon les revendications 1 et 2, **caractérisé en ce que** la turbulence régnant dans la zone de réaction correspond à un nombre de Reynolds d'au moins 2 500.

4. Procédé selon les revendications 1 à 3, **caractérisé en ce que** la température dans la zone de réaction va de 300 à 500°C.

5. Procédé selon les revendications 1 à 4, **caractérisé en ce que** le gaz inerte utilisé pour diluer la diamine à l'état de vapeurs, est l'azote.
